# EUROPÄISCHE PATENTANMELDUNG

(11) **EP 4 843 691 A1**
(43) Veröffentlichungstag der Anmeldung: **25.11.2026**
(21) Anmeldenummer: 26179242.8
(22) Anmeldetag: 18.05.2026
(51) Int. Cl.: B23D 47/00, B23Q 1/25, B27B 5/18, B27B 5/20, B27M 1/08, B23Q 3/00, B27G 3/00, G01N 1/28, G01N 33/46, G01N 35/00

(54) **VORRICHTUNG UND VERFAHREN ZUR PROBENPRÄPARATION**

(30) Priorität: 22.05.2025 DE 102025120056
(71) Anmelder: PUTZ INNOVA GmbH, 4822 Bad Goisern (AT)
(72) Erfinder: PUTZ, Stefan, 4822 Bad Goisern (AT)
(74) Vertreter: WSL Patentanwälte Partnerschaft mbB

(57) **Zusammenfassung**

Die Erfindung betrifft eine Vorrichtung zur Präparation von Proben, insbesondere von Proben aus Holzplatten wie OSB-, Span-, Verbund- oder Sperrholzplatten. Die Vorrichtung umfasst eine an einem Portal verfahrbare und absenkbare Bearbeitungseinrichtung mit einem Trennwerkzeug, das auf einer zu bearbeitenden Platte zu und hindurch beweglich geführt ist. Eine Staubabzugshaube ist an der Bearbeitungseinrichtung relativ zu dem Trennwerkzeug vertikal verschieblich gelagert und mit einem Niederhalter versehen, dessen umlaufende Stirnkante auf der Plattenoberfläche aufliegt. Die Bearbeitungseinrichtung ist mit einem Roboterarm koppelbar, der aus einem Werkzeugmagazin verschiedene Werkzeuge aufnehmen und das Bearbeitungswerkzeug entlang vordefinierter Schnittmuster führen kann. Die Erfindung betrifft ferner ein Verfahren zur Probenpräparation unter Verwendung der Vorrichtung, wobei die Probe automatisiert herausgetrennt, weiterbearbeitet und abgelegt wird.

## Beschreibung

Die Erfindung betrifft eine Vorrichtung und ein Verfahren zur Probenpräparation.

Es ist bekannt, dass insbesondere im Möbelbau, aber auch in anderen Bereichen der Holztechnik wie dem Hausbau, Sanierung, etc., Holz eingesetzt wird. Hierbei wird neben gewachsenem Holz auch sehr häufig Holz eingesetzt, welches zuvor zerspant wurde. Entsprechende Werkstoffe sind beispielsweise Sperrholzplatten, Spanplatten oder OSB-Platten. Um in der Fertigung derartiger Platten eine Qualitätsüberwachung durchführen zu können, ist es notwendig, aus diesen Platten Proben zu nehmen und diese Proben einer Untersuchung zuzuführen.

Derartige Untersuchungen sind beispielsweise Bruchversuche, Scherversuche, Zugversuche und Querzugversuche. Zusätzlich können insbesondere bei Spanplatten auch Versuche bezüglich des Ausdampfverhaltens bezüglich Formaldehyd durchgeführt werden.

Üblicherweise erfolgt die Probennahme dadurch, dass eine Platte aus der Fertigung entnommen wird, zerschnitten wird, auf ein geeignetes Maß zugeschnitten wird und dann der Beprobung zugeführt wird. Alle diese Schritte werden im Stand der Technik manuell durchgeführt. Dies verursacht hohe Kosten, hohen Verschnitt und kostet viel Zeit.

Aufgabe der Erfindung ist es, eine Vorrichtung zur Präparation von Proben aus den genannten Holzplatten zu schaffen, welche eine derartige Probenpräparation vollautomatisch, schnell, sicher, zuverlässig und günstig durchführt.

Die Aufgabe wird mit einer Vorrichtung mit den Merkmalen des Anspruchs 1 gelöst.

Vorteilhafte Weiterbildungen sind in Unteransprüchen gekennzeichnet.

Es ist eine weitere Aufgabe, ein Verfahren zur Probenpräparation zu schaffen, mit dem es gelingt, Proben zuverlässig, schnell, sicher und automatisiert zu erzeugen.

Die Aufgabe wird mit einem Verfahren mit den Merkmalen des Anspruchs 16 gelöst.

Vorteilhafte Weiterbildungen sind in den hiervon abhängigen Unteransprüchen gekennzeichnet.

Die erfindungsgemäße Vorrichtung ist so aufgebaut, dass eine Platte aus einer Produktion auf die Vorrichtung geschleust werden kann und dann innerhalb dieser Vorrichtung in definierter Lage festgelegt und gehalten wird.

Die Vorrichtung besitzt zur Probennahme ein verfahrbares Dreiachssystem, mit dem eine Bearbeitungseinrichtung an jeden Punkt der Platte verfahrbar ist und an diesem gewählten Punkt eine Bearbeitung durchführen kann.

Die Bearbeitung bedeutet in diesem Fall insbesondere, dass eine Probe einer gewünschten Ausdehnung aus der Platte ausgetrennt werden kann.

Die Trennung erfolgt hierbei insbesondere mittels eines Kreissägeblattes, eines Fingerfräsers, einer Stichsäge, einer Oszillationssäge, einem Laser oder einem Wasserstrahls oder einem anderen geeigneten Verfahren.

Insbesondere bei einer mechanischen Bearbeitung bzw. mechanischen Trennung ist die Vorrichtung so ausgebildet, dass über eine auf Längsschienen verfahrbaren Brücke eine Bewegung in X-Richtung über die Platte ermöglicht wird.

Die Brücke selber trägt die Bearbeitungseinrichtung, welche auf der Brücke in Y-Richtung verfahrbar ist.

Somit lässt sich jeder gewählte Punkt auf der Platte über die X- und Y-Verfahrrichtung ansteuern.

Insbesondere bei der mechanischen Bearbeitung ist es notwendig, das Bearbeitungswerkzeug der Bearbeitungseinrichtung auf die Platte abzusenken und zwar so weit, dass die Platte von dem Bearbeitungswerkzeug durchgriffen werden kann.

Erfindungsgemäß gelingt dies dadurch, dass das Bearbeitungswerkzeug linear an der Brücke verfahrbar angeordnet ist und insbesondere passiv verfahrbar angeordnet ist.

Die aktive Verfahrung des Bearbeitungswerkzeuges erfolgt über einen ohnehin vorhandenen Roboterarm, welcher während der Probenpräparation weitere Aufgaben hat. Erfindungsgemäß kann der Roboterarm das Bearbeitungswerkzeug gegen einen Federdruck auf die Platte drücken, sodass das Bearbeitungswerkzeug in eingeschaltetem Zustand entsprechend die Platte trennend durchgreift. Das Bearbeitungswerkzeug kann dann von dem Roboterarm zusammen mit der X- und Y-Verfahrung der Brücke entsprechend in der Platte verfahren werden, wenn dies notwendig ist.

Das Bearbeitungswerkzeug kann insbesondere, wenn eine Trennscheibe wie eine Sägescheibe verwendet wird, aber auch so ausgestaltet sein, dass die Sägescheibe so groß ist, dass sie nach dem Durchtrennen der Platte bereits die erforderliche Kantenlänge des Probenörpers erzeugt hat.

Ist auf diese Art und Weise ein Probenkörper, insbesondere ein quadratischer Probenkörper, aus der Platte ausgetrennt, kann der Roboterarm die Manipulation des Bearbeitungswerkzeuges einstellen und anschließend die Probe, beispielsweise mittels eines Sauggreifers, entnehmen und weiter verfahren.

Erfindungsgemäß ist am Bearbeitungswerkzeug eine Staubabsaughaube gleitend verschieblich angeordnet, dass die Staubabsaughaube beim Niederdrücken des Bearbeitungswerkzeuges in die Platte gegen einen Federdruck auf der Platte aufliegt, während das Bearbeitungswerkzeug in die Platte eintaucht.

Erfindungsgemäß hat sich herausgestellt, dass das Austrennen, insbesondere das automatisierte Heraustrennen dieser Probenkörper, nicht gelingt, wenn der Bereich des Probenkörpers, der herausgetrennt werden soll, nicht mit einem Niederhalter belastet wird.

Demgemäß wird nach der Erfindung ein Niederhalter verwendet, welcher während des Trennvorganges vom Trennwerkzeug räumlich entfernt, jedoch innerhalb des Probenkörperbereichs den Probenkörper gegen eine darunterliegende Platte oder Tischplatte belastet.

Diese Belastung kann durch den Niederhalter in Form eines elastischen Stiftes oder Stutzens erfolgen. Darüber hinaus kann die Belastung punktuell über einen metallischen Stift oder Stutzen erfolgen.

Bevorzugt wird bei der Erfindung jedoch eine bombierte Rolle eingesetzt, welche punktuell auf den Probenkörper einwirkt und dabei jedoch auch beweglich ist. Hierbei ist von Vorteil, dass bei einer Bewegung des Trennwerkzeuges oder des Bearbeitungswerkzeuges die Rolle dem folgen kann und den Probenkörper fortlaufend belastet.

Die Rolle kann hierbei insbesondere an der Absaughaube derart befestigt sein, dass sie über eine umlaufende Kante der Haube nach unten vorsteht.

Die Federkraft, gegen die die Haube auf der Platte aufsteht, wird hierbei auch dazu benutzt oder hauptsächlich dafür benutzt, den Niederhalter zu belasten. Zusätzlich wird hierbei ein gewisser geringer Abstand zwischen der umlaufenden Kante der Absaughaube und der Platte durch die Rolle gewährleistet, wobei dieser Abstand beispielsweise durch ein umlaufendes Bürstenelement überbrückt werden kann.

Der Roboterarm ist dabei so ausgebildet, dass beispielsweise bei einer quadratischen Probe mit einem Kreissägeblatt als Bearbeitungswerkzeug zunächst ein erster Schnitt durchgeführt wird, der Roboterarm das Bearbeitungswerkzeug dann um 90° dreht, ein weiterer Schnitt durchgeführt wird und nach zwei weiteren 90°-Drehungen der entsprechende Probenkörper ausgeschnitten ist.

Bei länglichen Probenkörpern muss gegebenenfalls entlang der langen Kanten das Bearbeitungswerkzeug verfahren werden.

Bei der Erfindung ist von Vorteil, dass in einfacher, nachvollziehbarer und sicherer Weise Probenkörper aus Holzplatten ausgetrennt werden können, um sie für eine weitere Präparation als Probenkörper zur Verfügung zu haben.

Die Erfindung betrifft insbesondere eine Vorrichtung zur Präparation von Proben, insbesondere von Proben aus Holzplatten, wie OSB-, Span-, Verbund- oder Sperrholzplatten, wobei eine an einem Portal verfahrbare und absenkbare Bearbeitungseinrichtung vorhanden ist, welche ein Trennwerkzeug aufweist, wobei das Trennwerkzeug mit der Bearbeitungseinrichtung auf einer zu bearbeitenden Platte zu und hindurch beweglich ausgebildet ist und wobei eine Staubabzugshaube vorgesehen ist, welche an der Bearbeitungseinrichtung relativ zu einem Bearbeitungswerkzeug vertikal verschieblich gelagert ist, wobei an der Staubabzugshaube ein Niederhalter angeordnet ist, welcher über eine untere, umlaufend an einer zu bearbeitenden Platte anliegende Stirnkante übersteht und horizontal beabstandet zu dem Bearbeitungswerkzeug angeordnet ist.

Eine Weiterbildung sieht vor, dass die Plattform eine horizontale Oberseite zur Aufnahme eines Roboterarms, insbesondere eines hyperredundanten Manipulators, und eine vertikale Vorderseite zur Aufnahme der Bearbeitungseinrichtung aufweist.

Eine Weiterbildung sieht vor, dass die Bearbeitungseinrichtung auf einer Linearführung vertikal verschieblich gelagert ist und über eine durch eine Feder gehaltene, abgehobene Nullposition verfügt, gegen deren Federkraft sie auf eine zu bearbeitende Platte abgesenkt werden kann.

Eine Weiterbildung sieht vor, dass die Bearbeitungseinrichtung um eine Drehachse drehbar gelagert ist und über ein Kopplungselement verfügt, welches eine drehfeste Verbindung mit dem Roboterarm ermöglicht.

Eine Weiterbildung sieht vor, dass der Roboterarm an einem freien Ende über eine Kopplungseinrichtung verfügt, mit der er unterschiedliche Werkzeuge, insbesondere aus einem Werkzeugmagazin, aufnehmen und mit der Bearbeitungseinrichtung koppeln kann.

Eine Weiterbildung sieht vor, dass die Staubabzugshaube aus mehreren ineinander verschiebbaren Teilen besteht und bei Druck auf den Niederhalter infolge des Absenkens des Bearbeitungswerkzeugs in die zu beprobende Platte eine definierte Abstandshöhe zur Platte gewährleistet.

Eine Weiterbildung sieht vor, dass der Niederhalter als eine bombierte Rolle oder als ein gleitender Niederhalter aus Kunststoff, Metall oder Keramik ausgebildet ist.

Eine Weiterbildung sieht vor, dass das Bearbeitungswerkzeug ein Kreissägeblatt, eine Oszillationssäge, ein Fräser, Laser oder Wasserstrahl ist.

Eine Weiterbildung sieht vor, dass innerhalb der Staubabzugshaube das Bearbeitungswerkzeug, insbesondere ein Kreissägeblatt, auf einer Welle eines Antriebs drehbar gelagert ist.

Eine Weiterbildung sieht vor, dass ein rotierbares Führungsrohr an seinem unteren Ende luftleitend mit der Staubabzugshaube verbunden ist und im Bereich seines diametral gegenüberliegenden, oberen Endes einen Schleifring und Durchbrechungen aufweist, welche mit einem Abluftkanal verbunden sind.

Eine Weiterbildung sieht vor, dass von dem Abluftkanal eine Abluftleitung durch die Brücke und in Dichtungskanäle des Portals geführt ist.

Eine Weiterbildung sieht vor, dass auf einer Arbeitsfläche unterhalb des Portals die zu beprobende Holzplatte positionierbar und durch Vakuum oder mechanische Niederhalter fixierbar ist.

Eine Weiterbildung sieht vor, dass der Roboterarm über eine wechselbare Greifvorrichtung oder Ansaugeinrichtung verfügt, mit der er Proben manipulieren, transportieren und ein oder beidseitig an Bearbeitungsstationen bearbeiten kann.

Eine Weiterbildung sieht vor, dass ein Druckwerkzeug die Proben mit einer eindeutigen Kennzeichnung beschriftet.

Eine Weiterbildung sieht vor, dass der Roboterarm freigeschnittene Proben in ein Probenmagazin ablegt, welches manuell oder automatisch zur Weiterverarbeitung bereitgestellt oder überführt wird.

Die Erfindung betrifft ferner ein Verfahren zur Präparation von Proben, insbesondere von Proben aus Holzplatten wie OSB-, Span-, Verbund- oder Sperrholzplatten, wobei eine zu beprobende Platte auf einer unterhalb des Portals angeordneten Arbeitsfläche positioniert und optional fixiert wird, wobei die Plattform mit dem Roboterarm und der Bearbeitungseinrichtung zu einem vordefinierten Beprobungspunkt verfahren wird.

Eine Weiterbildung sieht vor, dass der Roboterarm mit der Bearbeitungseinrichtung gekoppelt wird und das Bearbeitungswerkzeug durch den Antrieb in Rotation versetzt wird.

Eine Weiterbildung sieht vor, dass die Bearbeitungseinrichtung durch den Roboterarm abgesenkt wird, bis der Niederhalter auf der Platte aufliegt und das Bearbeitungswerkzeug in die Platte eindringt, um mit dem Portal entlang eines definierten Schnittmusters einen Trennschnitt auszuführen.

Eine Weiterbildung sieht vor, dass der Roboterarm die Bearbeitungseinrichtung nach Beendigung des Trennschrittes anhebt, neu positioniert und weitere Trennschnitte ausführt, bis die Probe vollständig freigeschnitten ist.

Eine Weiterbildung sieht vor, dass der Roboterarm aus einem Werkzeugmagazin ein Werkzeug zur Aufnahme der freigeschnittenen Probe koppelt, die Probe aufnimmt, weiterverarbeitet, insbesondere zur Oberflächen- und Kantenbearbeitung sowie zur Bedruckung, und in einem Magazin oder an einem Ablageort ablegt.

Die Erfindung wird anhand einer Zeichnung beispielhaft erläutert. Es zeigen dabei:

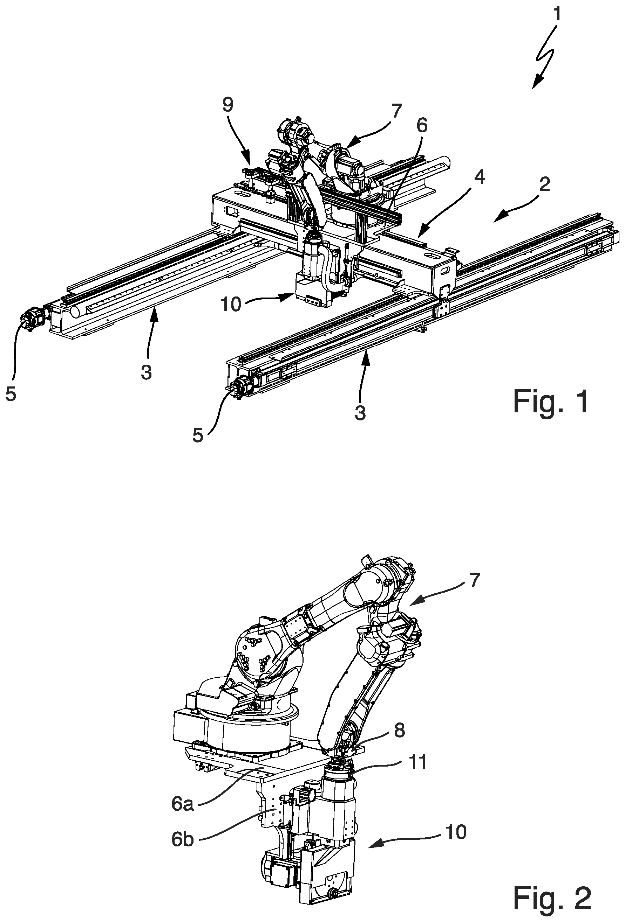

- **Fig. 1:**: eine isometrische Ansicht auf die Vorrichtung mit den Längsschienen und der Brücke und den daran angeordneten Aggregaten;
- **Fig. 2:**: den an der Brücke angeordneten Roboterarm, gekoppelt mit dem Bearbeitungswerkzeug;
- **Fig. 3:**: das Bearbeitungswerkzeug in einer isometrischen Ansicht;
- **Fig. 4:**: das Bearbeitungswerkzeug nach Figur 3 in einer weiteren isometrischen Ansicht;
- **Fig. 5:**: das Bearbeitungswerkzeug in einer weiteren isometrischen Ansicht;
- **Fig. 6:**: das Bearbeitungswerkzeug in einer isometrischen Ansicht von unten;
- **Fig. 7:**: das Bearbeitungswerkzeug, insbesondere zeigend die Absaughaube in einer weiteren isometrischen Ansicht von unten;
- **Fig. 8:**: das Bearbeitungswerkzeug in einem Längsschnitt parallel zu einem Kreissägeblatt;
- **Fig. 9:**: einen weiteren Querschnitt durch das Bearbeitungswerkzeug.

Die erfindungsgemäße Vorrichtung 1 zur Präparation von Proben aus Holzplatten besitzt ein Portal 2, wobei das Portal 2 zwei parallel zueinander verlaufende sich längst erstreckende Führungsschienen 3 besitzt, auf denen eine Brücke 4 in an sich bekannter Weise verschieblich gelagert ist.

Die Brücke 4 ist dabei insbesondere motorisch durch Spindelmotoren, Schrittmotoren, Linearmotoren 5 oder dergleichen auf den Führungsschienen 3 beweglich. Auf der Brücke 4 ist eine beweglich verschiebliche Plattform 6 angeordnet, welche auf der Brücke 4 zwischen den Führungsschienen 3 verschieblich ist.

Die Plattform 6 kann insbesondere auch über Schrittmotoren, Linearmotoren, Spindelmotoren 5 oder dergleichen an sich bekannte Bewegungseinrichtungen auf der Brücke 4 beweglich angeordnet sein. Die Plattform 6 besitzt eine horizontale Oberseite 6A und eine vertikale plattenartige Vorderseite 6B.

Auf der plattenartigen horizontalen Oberseite 6A ist ein an sich bekannter Roboterarm 7 angeordnet, welcher in der Lage ist, neben 360°-Drehungen auch Auf- und Abbewegungen und Bewegungen weg von seiner Drehachse durchzuführen. Derartige Roboterarme 7 sind an sich bekannt.

Der Roboterarm 7 besitzt an einem freien Ende 8 insbesondere Kopplungseinrichtungen, mit denen er an unterschiedliche Werkzeuge derart ankoppeln kann, dass diese durch ihn über eine zu bearbeitende Arbeitsfläche bewegt werden können und gegenüber der Arbeitsfläche auch abgesenkt werden können und gegebenenfalls auch Tätigkeiten wie Ansaugen, Abstoßen und ähnliches durchführen können.

Für derartige Betätigungswerkzeuge kann ein Betätigungswerkzeugmagazin 9 an der Brücke 4 oder der Plattform 6 angeordnet sein.

Die vertikale Vorderseite 6B trägt die Bearbeitungseinrichtung 10.

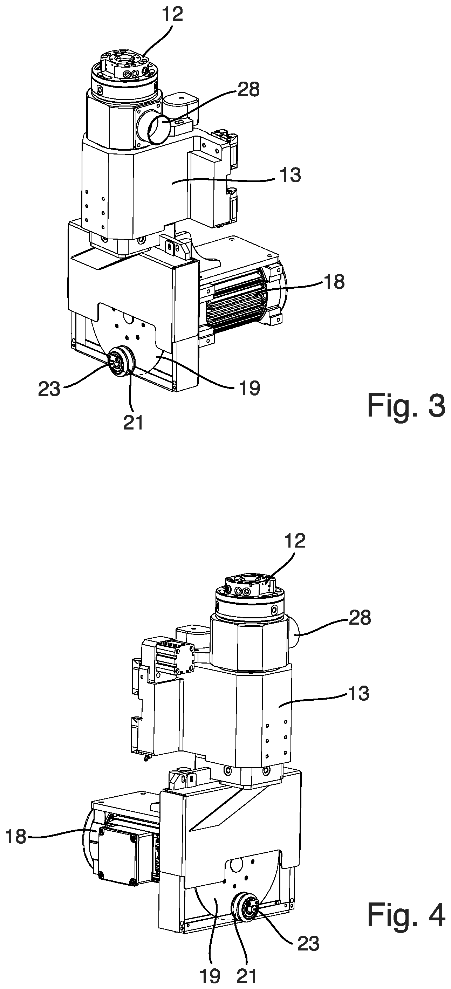

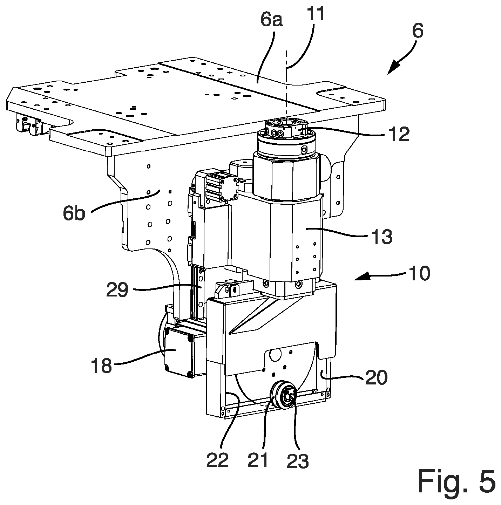

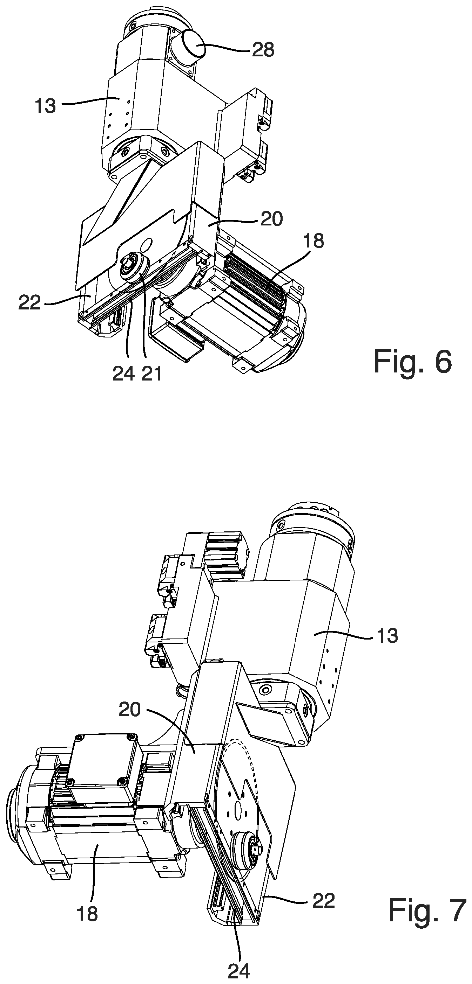

Die Bearbeitungseinrichtung 10 ist insbesondere in den Figuren 3 bis 9 dargestellt. Mit Bezug zunächst auf Figur 5 ist ersichtlich, dass die Bearbeitungseinrichtung 10 an der vertikalen Vorderseite 6B der Plattform 6 angeordnet ist. Hierbei ist die Bearbeitungseinrichtung 10 an der Platte 6B vertikal verschieblich gelagert, wobei die, von einer zu bearbeitenden Platte abgehobene Nullposition der Bearbeitungseinrichtung 10 durch Federdruck gehalten wird. Die Bearbeitungseinrichtung 10 ist um eine Drehachse 11 um 360° drehbar ausgebildet, wobei die Bearbeitungseinrichtung 10 ein Kopplungselement 12 besitzt, an welches eine Gegenkopplungseinrichtung des Roboterarms 7 ankoppelbar ist. Hierdurch wird es ermöglicht, dass der Roboter die Bearbeitungseinrichtung 10 um 360° drehen kann.

Zudem kann der Roboterarm 7 die Bearbeitungseinrichtung 10 gegen den Druck der Feder (nicht gezeigt) auf die zu bearbeitende Platte drücken.

Die Bearbeitungseinrichtung 10 besitzt ein oberes Traggehäuse 13, mit dem die Bearbeitungseinrichtung 10 an der Vorderseite 6B über Linearführungen 29 vertikal verschieblich angeordnet ist. Innerhalb des oberen Traggehäuses ist die Bearbeitungseinrichtung 10 mit einem Führungsrohr 14 drehbar gelagert. Innerhalb des oberen Traggehäuses ist auch die Feder 15 angeordnet, gegen welche die Trageinrichtung vertikal verschieblich ist. Unterhalb des oberen Traggehäuses ist an einem freien Ende 16 des Führungsrohres 14 eine Tragwandung 17 angeordnet, welche einerseits einen motorischen Antrieb 18 für ein Bearbeitungswerkzeug 19 trägt und andererseits eine hiergegen federnd gelagerte Staubabzugshaube 20 trägt, welche gegen die Tragwandung 17 federnd gelagert ist. Dem Antrieb 18 diametral gegenüberliegend ist an der verschieblich gelagerten Staubabzugshaube der Niederhalter 21 drehbar gelagert. Der Niederhalter 21 ist bei dieser Ausführung eine bombierte Rolle 21, deren Drehwelle 23 horizontal von einer Wandung 22 der Staubabzugshaube absteht, wobei die Drehwelle 23 dabei derart an der Wandung 22 der Staubabzugshaube 20 angeordnet ist, dass die bombierte Rolle mit einem vorbestimmten Betrag über eine untere umlaufende Kante 24 der Staubabzugshaube 20 vorsteht. Der Niederhalter 21 generell und die bombierte Rolle 21 im Speziellen ist dabei so angeordnet, dass der Auflagepunkt des Niederhalters bzw. der bombierten Rolle 21 auf einer Platte exakt mit der Drehachse 11 des Führungsrohres 14 zusammenfällt. Hierdurch ist gewährleistet, dass bei einer Drehung des Führungsrohres 14 um die Drehachse 11 der Niederhalter 21 auf dem Punkt mitgedreht wird.

Innerhalb der Staubabzugshaube 20 ist das Bearbeitungswerkzeug 19 auf einer Drehwelle 18A des Antriebs 18 drehbar angeordnet. Der Abstand des Bearbeitungswerkzeuges 19 und insbesondere eines Kreissägeblattes vom Auflagepunkt des Niederhalters 21 entspricht einer halben Probenbreite. Werden andere Probendimensionen gewünscht, ist somit insbesondere der Abstand des Bearbeitungswerkzeug 19, insbesondere eines Kreissägeblattes zum Niederhalter 21 zu vergrößern, da der Niederhalter 21 in jedem Fall mit der Drehachse 11 fluchten sollte.

Nachfolgend soll die Arbeitsweise kurz erläutert werden.

Zu Beginn eines Arbeitszyklus wird eine zu beprobende Holzplatte auf einen Bearbeitungstisch verbracht, der vom erfindungsgemäßen Portal 2 überbaut ist. Anschließend kann mit dem Portal 2 jeder beliebige Punkt der Platte angefahren werden. Üblicherweise sind die Probennahmepunkte vorher bestimmt und genügen insbesondere statistischen Anforderungen an eine Beprobung.

Der Roboterarm 7 kann bereits mit der Bearbeitungseinrichtung 10 gekoppelt sein oder wird mit der Bearbeitungseinrichtung 10 gekoppelt, sobald der Beprobungspunkt erreicht wird. Anschließend wird der Antrieb 18 in Gang gesetzt, sodass das Bearbeitungswerkzeug 19, insbesondere ein Kreissägeblatt in Drehung versetzt wird. Um nun eine Probe herauszuschneiden, wird das Portal so verfahren, dass der Niederhalter 21 mit seinem Auflagepunkt sich oberhalb des Mittelpunkts der Probe befindet. Anschließend drückt der Roboterarm 7 die Bearbeitungseinrichtung 10 in Richtung zur Platte, bis der Niederhalter 21 auf der Platte aufliegt. Bei weiterem definierten Verdrücken der Bearbeitungseinrichtung 10 auf die Platte wird die Staubabzugshaube 20 sich nicht weiter an die Oberfläche der Platte annähern, da der Abstand durch den Überstand des Niederhalters 21 eingestellt wird. Jedoch gelangt das Kreissägeblatt gegen den Druck der Feder 15 in die zu bearbeitende Platte und wird dabei vorzugsweise so weit eingedrückt, dass das Kreissägeblatt insbesondere auf der gesamten Länge der gewünschten Schnittkante die zu bearbeitende Platte durchdringt.

Nachdem hiermit der erste Schnitt getätigt ist, hebt der Roboterarm 7 die Bearbeitungseinrichtung 10 wieder so weit an, dass das Kreissägeblatt aus der Platte herausgelangt. Nun dreht der Roboterarm 7 die Bearbeitungseinrichtung 10 insbesondere um 90° und wiederholt den Schnittvorgang. Nach zwei weiteren 90°-Drehungen sind alle vier Schnittkanten eines Quadrats geschnitten und durch eine entsprechende Schnittüberlappung sichergestellt, dass die Probe aus der Platte freigeschnitten ist.

Selbstverständlich müssen keine nachfolgenden oder aufeinanderfolgenden 90°-Drehungen erfolgen, es können auch zwei 180°-Drehungen und eine dazwischenliegende 90°-Drehung vorgesehen werden oder jedes andere Drehmuster.

Beim Freischneiden ist wesentlich, dass ein Niederhalter 21 vorhanden ist, der wie zuvor beschrieben aus einer bombierten Rolle bestehen kann. Eine bombierte Rolle hat den Vorteil, dass bei nicht quadratischen Schnitten, sondern insbesondere länglich leistenartigen Schnitten, der Niederhalter 21 einer fortlaufenden Schneidbewegung folgen kann. Bei lediglich quadratischen Schnitten könnte an sich auch ein nicht als Rolle ausgebildeter gleitender Niederhalter 21 mit einer PTFE-Beschichtung oder aus einem abgerundeten Stahl oder Kunststoff ausreichend sein.

Bevorzugt ist, dass der Niederhalter 21 eine Kraft derart auf die Probe aufbringt, dass sie bei dem Schneiden nicht verrutscht, da sich hierdurch insbesondere ausgefranzte Ecken ergeben können, welche nachteilig sind.

Anstelle eines Kreissägeblattes ist selbstverständlich auch jedes andere Trennmittel möglich, insbesondere auch punktuelle Trennmittel. Derartige punktuelle Trennmittel können z.B. Fingerfräser, oszillierende Werkzeuge, Laser, Wasserschneider und andere Werkzeuge oder Einrichtungen sein. Bei punktueller Einwirkung muss selbstverständlich die gesamte Schnittlinie durch das Portal abgefahren werden.

Das erfindungsgemäße Verfahren sieht zudem vor, dass die Proben aus der Platte nach einem festgelegten Schnittmuster in einheitlichen oder unterschiedlichen Größen herausgeschnitten werden. Hierfür wird die zu bearbeitende Platte wie bereits ausgeführt unterhalb des Portals auf eine Arbeitsfläche aufgelegt und vorzugsweise auf dieser Arbeitsfläche mit Anschlägen in eine definierte Position gebracht.

Diese definierte Position kann anschließend durch insbesondere Ansaugen mit Vakuum oder Aufdrücken mit entsprechenden Niederhaltern 21 generell gehalten werden.

Auf dem Bearbeitungstisch kann gegebenenfalls eine sogenannte Opferplatte angeordnet sein, welche nach einer Mehrzahl von Beprobungsvorgängen ausgetauscht werden kann, wenn zu viele Schnitte darauf angeordnet sind. Eine solche Opferplatte kann insbesondere derart luftdurchlässig ausgebildet sein, dass durch die Opferplatte ein Vakuum angesaugt werden kann.

Die Vorrichtung zur Probenpräparation kann zudem Einrichtungen zur Oberflächenbearbeitung aufweisen. Insbesondere kann der Roboterarm 7 hierfür Schleif-Fräs-Einrichtungen wie Schleifscheiben als zusätzliche Werkzeuge aufnehmen sowie eine Schleif-Fräs-Einrichtungen in der Reichweite des Roboterarms 7 angeordnet sein und damit sowohl die Breitseiten der Proben als auch die Schnittkanten der Proben nachbearbeiten.

Zudem kann ein Versiegelungswerkzeug vorhanden sein, mit dem die Schnittkanten insbesondere für Saugproben entweder mit einem Klebeband, mit einer Klebeschicht oder dergleichen versiegelt werden können, um ein vertikales Saugen durch die Plattenfläche der Proben zu gewährleisten.

Die Vorrichtung kann zudem über eine Druckvorrichtung verfügen, welche jede Probe mit einer eindeutigen Probenbezeichnung versieht, um die Proben später für nachvollziehbare Nachversuche zurückzustellen.

Darüber hinaus kann es vorteilhaft sein, an der Vorrichtung für die Proben Probenhalter bzw. Probenmagazine vorzusehen, in die eine Mehrzahl von Proben aufeinandergestapelt abgelegt werden können, wobei die Probenmagazine nach Auffüllung an die eigentliche Beprobungsstation weitergegeben werden können.

Die Weitergabe an eine Beprobungsstation kann hierbei vollautomatisch erfolgen oder die Probenmagazine werden an einer entsprechenden Station zur Abholung bereitgestellt.

Darüber hinaus können an der Vorrichtung unterschiedliche Bearbeitungsstationen angeordnet sein, wobei der Roboterarm 7 über einen Greifer oder eine Ansaugvorrichtung verfügt, mit der er Proben ansaugen kann und entsprechend an die Bearbeitungsstationen halten kann.

Darüber hinaus kann im Bereich der Bearbeitungsstationen eine Ablage vorhanden sein, mit der es gelingt, dass der Roboterarm 7 die Proben nach Drehung auch an der gegenüberliegenden Seite bearbeitet.

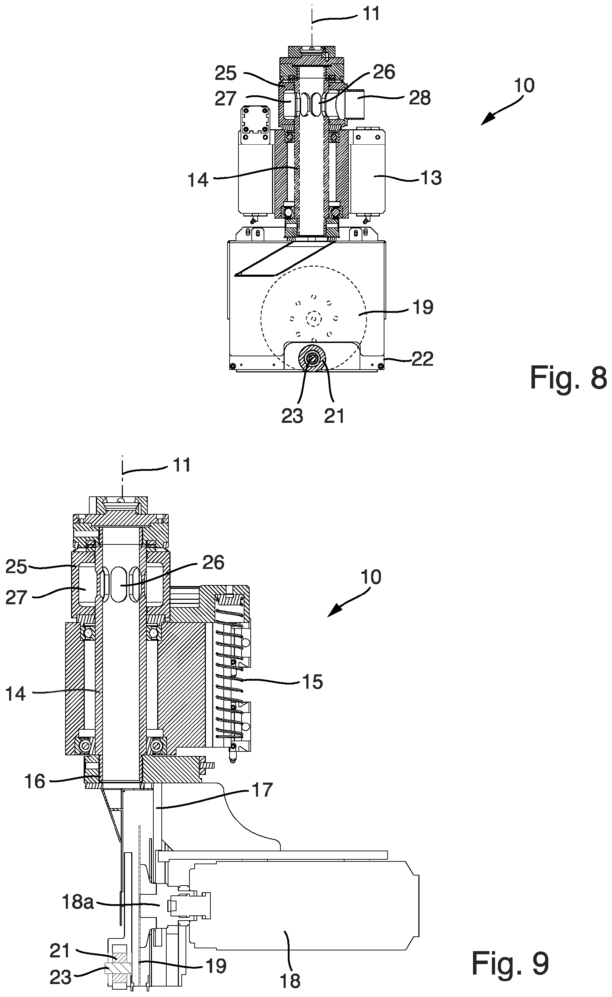

Vorteilhaft ist (Figuren 8, 9), wenn das Führungsrohr 14 an seinem unteren freien Ende mit der Staubabsaughaube luftleitend verbunden ist und benachbart zu dem Kopplungsbereich oben ein luftführender Schleifring derart vorhanden ist, dass das Führungsrohr 14 innerhalb des Schleifrings 25 frei drehbar ist. Hierzu besitzt das Führungsrohr 14 im Bereich des Schleifrings 25 Durchbrechungen 26, sodass der luftleitende Innenraum des Führungsrohrs 14 in den luftführenden Innenraum 27 des Schleifrings mündet, sodass der Staub durch das Führungsrohr 14 in den Schleifring 25 und vom Schleifring 25 in eine Abluftleitung 28 gesaugt werden kann. Hierbei ist von Vorteil, dass dies eine vergleichsweise einfache, aber effektive Konstruktion einer Staubabsaugung gewährleistet, ohne dass irgendwelche luftführenden flexiblen Schläuche vorhanden sein müssten. Die Abluftleitung 28 kann hierbei in die Brücke geführt werden und von der Brücke entsprechend in über Dichtlippen gedichtete Kanäle des Portals bzw. der Längsträger. Derartige Ausgestaltungen sind an sich bekannt.

In einer entsprechenden Konstruktion der Plattform 6 fährt die Bearbeitungseinrichtung 10 immer passiv auch mit dem Roboterarm 7 mit, sodass dieser jederzeit auch an der Bearbeitungseinrichtung 10 ankoppeln und diese bewegen kann, sofern der Roboter nicht andere Aufgaben erledigt.

Bei der Erfindung ist von Vorteil, dass es gelingt, in vergleichsweise einfacher, jedoch sehr robuster Art und Weise eine automatisierte Probenpräparation zu erzielen, mit welcher Proben nicht nur aus Holzplatten ausgeschnitten werden können, sondern gegebenenfalls über einen Roboterarm 7 auch einer weiteren Probenpräparation wie Kantenschleifen, Schnittflächenschleifen, Oberflächenschleifen, Kantenversiegelung und dergleichen zugeführt werden können.

### Liste der Bezugszeichen

1. Vorrichtung
2. Portal
3. Führungsschiene
4. Brücke
5. Spindelmotor
6. Plattform
   6a. Horizontale Oberseite der Plattform
   6b. Vertikale plattenartige Vorderseite
7. Roboterarm
8. Freies Ende
9. Werkzeugmagazin
10. Bearbeitungseinrichtung
11. Drehachse
12.Kopplungselement
13. Oberes Traggehäuse
14. Führungsrohr
15. Feder
16. freies Ende
17.Tragwandung
18. Antrieb
   18a. Drehwelle
19. Bearbeitungswerkzeug
20. Staubabzugshaube
21. Niederhalter
22.Wandung
23. Drehbare Welle
24. Kante
25. Schleifring
26. Durchbrechung
27. Innenraum
28.Abluftleitung
29. Linearführungen

## Patentansprüche

1. Vorrichtung zur Präparation von Proben, insbesondere von Proben aus Holzplatten, wie OSB-, Span-, Verbund- oder Sperrholzplatten, wobei eine an einem Portal (2) verfahrbare und absenkbare Bearbeitungseinrichtung (10) vorhanden ist, welche ein Trennwerkzeug (19) aufweist, wobei das Trennwerkzeug (19) mit der Bearbeitungseinrichtung (10) auf einer zu bearbeitenden Platte zu und hindurch beweglich ausgebildet ist und wobei eine Staubabzugshaube (20) vorgesehen ist, welche an der Bearbeitungseinrichtung (10) relativ zu einem Bearbeitungswerkzeug (19) vertikal verschieblich gelagert ist, wobei an der Staubabzugshaube (20) ein Niederhalter (21) angeordnet ist, welcher über eine untere, umlaufend an einer zu bearbeitenden Platte anliegende Stirnkante (24) übersteht und horizontal beabstandet zu dem Bearbeitungswerkzeug (19) angeordnet ist, wobei eine Plattform (6) eine horizontale Oberseite (6a) zur Aufnahme eines Roboterarms (7), insbesondere eines hyperredundanten Manipulators, und eine vertikale Vorderseite (6b) zur Aufnahme der Bearbeitungseinrichtung (10) aufweist.

2. Vorrichtung nach einem der vorhergehenden Ansprüche, **dadurch gekennzeichnet, dass** die Bearbeitungseinrichtung (10) auf einer Linearführung (29) vertikal verschieblich gelagert ist und über eine durch eine Feder (15) gehaltene, abgehobene Nullposition verfügt, gegen deren Federkraft sie auf eine zu bearbeitende Platte abgesenkt werden kann.

3. Vorrichtung nach einem der vorhergehenden Ansprüche, **dadurch gekennzeichnet, dass** die Bearbeitungseinrichtung (10) um eine Drehachse (11) drehbar gelagert ist und über ein Kopplungselement (12) verfügt, welches eine drehfeste Verbindung mit dem Roboterarm (7) ermöglicht.

4. Vorrichtung nach einem der vorhergehenden Ansprüche, **dadurch gekennzeichnet, dass** der Roboterarm (7) an einem freien Ende (8) über eine Kopplungseinrichtung (12) verfügt, mit der er unterschiedliche Werkzeuge, insbesondere aus einem Werkzeugmagazin (9), aufnehmen und mit der Bearbeitungseinrichtung (10) koppeln kann.

5. Vorrichtung nach einem der vorhergehenden Ansprüche, **dadurch gekennzeichnet, dass** die Staubabzugshaube (20) aus mehreren ineinander verschiebbaren Teilen besteht und bei Druck auf den Niederhalter (21) infolge des Absenkens des Bearbeitungswerkzeugs (19) in die zu beprobende Platte eine definierte Abstandshöhe zur Platte gewährleistet.

6. Vorrichtung nach einem der Vorherigen Ansprüche, **dadurch gekennzeichnet, dass** der Niederhalter (21) als eine bombierte Rolle oder als ein gleitender Niederhalter aus Kunststoff, Metall oder Keramik ausgebildet ist.

7. Vorrichtung nach einem der vorhergehenden Ansprüche, **dadurch gekennzeichnet, dass** das Bearbeitungswerkzeug (19) ein Kreissägeblatt, eine Oszillationssäge, ein Fräser, Laser oder Wasserstrahl ist.

8. Vorrichtung nach einem der vorhergehenden Ansprüche, **dadurch gekennzeichnet, dass** innerhalb der Staubabzugshaube (20) das Bearbeitungswerkzeug (19), insbesondere ein Kreissägeblatt, auf einer Welle (18a) eines Antriebs (18) drehbar gelagert ist.

9. Vorrichtung nach einem der vorhergehenden Ansprüche, **dadurch gekennzeichnet, dass** ein rotierbares Führungsrohr (14) an seinem unteren Ende luftleitend mit der Staubabzugshaube (20) verbunden ist und im Bereich seines diametral gegenüberliegenden, oberen Endes einen Schleifring (25) und Durchbrechungen (26) aufweist, welche mit einem Abluftkanal (28) verbunden sind.

10. Vorrichtung nach einem der vorhergehenden Ansprüche, **dadurch gekennzeichnet, dass** von dem Abluftkanal (28) eine Abluftleitung durch die Brücke (4) und in Dichtungskanäle des Portals (2) geführt ist.

11. Vorrichtung nach einem der vorhergehenden Ansprüche, **dadurch gekennzeichnet, dass** auf einer Arbeitsfläche unterhalb des Portals (2) die zu beprobende Holzplatte positionierbar und durch Vakuum oder mechanische Niederhalter fixierbar ist.

12. Vorrichtung nach einem der vorhergehenden Ansprüche, **dadurch gekennzeichnet, dass** der Roboterarm (7) über eine wechselbare Greif- oder Ansaugeinrichtung verfügt, mit der er Proben manipulieren, transportieren und ein oder beidseitig an Bearbeitungsstationen bearbeiten kann.

13. Vorrichtung nach einem der vorhergehenden Ansprüche, **dadurch gekennzeichnet, dass** ein Druckwerkzeug die Proben mit einer eindeutigen Kennzeichnung beschriftet.

14. Vorrichtung nach einem der vorhergehenden Ansprüche, **dadurch gekennzeichnet, dass** der Roboterarm (7) freigeschnittene Proben in ein Probenmagazin ablegt, welches manuell oder automatisch zur Weiterverarbeitung bereitgestellt oder überführt wird.

15. Verfahren zur Präparation von Proben, insbesondere von Proben aus Holzplatten wie OSB-, Span-, Verbund- oder Sperrholzplatten, unter Verwendung einer Vorrichtung nach Anspruch 1 - 14, wobei eine zu beprobende Platte auf einer unterhalb des Portals (2) angeordneten Arbeitsfläche positioniert und optional fixiert wird, wobei die Plattform (6) mit dem Roboterarm (7) und der Bearbeitungseinrichtung (10) zu einem vordefinierten Beprobungspunkt verfahren wird.

16. Verfahren nach Anspruch 15, **dadurch gekennzeichnet, dass** der Roboterarm (7) mit der Bearbeitungseinrichtung (10) gekoppelt wird und das Bearbeitungswerkzeug (19) durch den Antrieb (18) in Rotation versetzt wird.

17. Verfahren nach Anspruch 15 und 16, **dadurch gekennzeichnet, dass** die Bearbeitungseinrichtung (10) durch den Roboterarm (7) abgesenkt wird, bis der Niederhalter (21) auf der Platte aufliegt und das Bearbeitungswerkzeug (19) in die Platte eindringt, um mit dem Portal (2) entlang eines definierten Schnittmusters einen Trennschnitt auszuführen.

18. Verfahren nach Anspruch 15 - 17, **dadurch gekennzeichnet, dass** der Roboterarm (7) die Bearbeitungseinrichtung (10) nach Beendigung des Trennschrittes anhebt, neu positioniert und weitere Trennschnitte ausführt, bis die Probe vollständig freigeschnitten ist.

19. Verfahren nach Anspruch 15 - 18, **dadurch gekennzeichnet, dass** der Roboterarm (7) aus einem Werkzeugmagazin (9) ein Werkzeug zur Aufnahme der freigeschnittenen Probe koppelt, die Probe aufnimmt, weiterverarbeitet, insbesondere zur Oberflächen- und Kantenbearbeitung sowie zur Bedruckung, und in einem Magazin oder an einem Ablageort ablegt.
